# EUROPEAN PATENT SPECIFICATION

(11) **EP 0 317 243 B1**
(45) Date of publication and mention of the grant of the patent: **31.08.1994**
(21) Application number: 88310736.9
(22) Date of filing: 14.11.1988
(51) Int. Cl.: C12Q 1/00, C12Q 1/26, C12Q 1/52, C12Q 1/56, C12Q 1/42, C12Q 1/40

(54) **Method and test composition for determination of enzyme activity**
Verfahren und Testzusammensetzung zur Bestimmung von Enzym-Aktivität
Procédé et composé de test pour la détermination de l'activité d'enzyme

(30) Priority: 13.11.1987 JP 286559/87
(43) Date of publication of application: 24.05.1989
(73) Proprietor: Kyowa Medex Co. Ltd., Tokyo (JP)
(72) Inventor: Miike, Akira, Sunto-gun Shizuoka-ken (JP); Tatano, Toshio, Numazu-shi Shuzuoka-ken (JP)
(74) Representative: Lambert, Hugh Richmond

(56) References cited:
- EP-A- 0 152 274
- FR-A- 2 567 907
- GB-A- 2 162 946
- US-A- 4 681 841

## Description

The present invention relates to a method and test composition for determination of an enzyme activity, and more particularly to a method for determination of an activity of an enzyme contained in a living body. The method comprises adding an appropriate substrate for an enzyme to a living body sample to thereby form a reaction product, which is capable of accelerating a rate of a reaction in which a chromogen is oxidised by an oxidase in the presence of oxygen; oxidising the chromogen by the oxidase in the presence of that reaction product and oxygen to form a pigment; and quantitatively determining the pigment in a conventional manner. The present invention also pertains to a test composition suitable for carrying out such determination.

The enzymes to which the present invention is applicable include, for example, γ-glutamyl transpeptidase (γ-GTP), leucine aminopeptidase (LAP), alanine aminopeptidase (AAP), cystine aminopeptidase (CAP), blood coagulation factor X, thrombin, plasmin, kallikrein, chymotrypsin, alkali phosphatase, N-acetyl glucosaminase and amylase.

γ-GTP, LAP and alkali phosphatase reflect troubles of biliary and hepatic organs. AAP and N-acetyl glucosaminase are an indicator for renal disorder. CAP is an indicator for movement in the uterus of a pregnant woman. Coagulation factor X, thrombin and plasmin are involved in the coagulation of blood. Kallikrein is an indicator for primary aldosteronism and hypertension. Chymotrypsin is an indicator for chronic pancreatitis. Amylase is an indicator for pancreatic disorder.

Until now the determination of the activity of γ-GTP, LAP and AAP has been carried out by adding a substrate for the enzyme to a sample containing the enzyme, further adding a necessary enzyme for deriving an analysable substance from the substrate, if necessary, and measuring the rate of formation of a resulting measurable compound. For example, the activity of γ-GTP or LAP is quantitatively determined by decomposing an appropriate substrate for γ-GTP or LAP by the enzyme, thereby forming an aniline derivative, allowing the aniline derivative to react with a chromogen to thereby form a pigment, and quantitatively determining the rate of formation of the pigment. When the amount of the enzyme is small, a chromogen capable of forming a pigment having a high molecular extinction coefficient is used, but such a conventional method has a limit in determination of an activity of a trace amount of enzyme.

As prior art mention may be made of FR-A-2567907 and US-A-4681841. Each discloses an enzyme assay procedure in which, in a first enzymatic reaction an aniline derivative is liberated from a synthetic substrate by the enzyme under assay. That aniline derivative is then oxidatively condensed with a coupler by the action of a second enzyme, viz. an oxidase, resulting in the formation of a pigment. By contrast, the present invention utilises a substrate or synthetic substrate which liberates an aniline derivative that merely catalyses the enzymatic oxidation of a chromogen with an oxidase to form a pigment, without the formation of a condensation product with the chromogen. In other words, the aniline derivative merely acts as an activator and enhances the activity of the oxidase, thus increasing the rate at which the pigment is produced, that rate being proportional to the activity of the first enzyme.

In accordance with the present invention, therefore, a method for the chromogenic determination of the activity of an enzyme in a sample is provided, which comprises reacting the sample with a substrate for that enzyme and capable of reacting with the enzyme to produce an aniline derivative in an amount proportional to the activity of the enzyme, enzymatically oxidising a chromogen by reaction with an oxidase in the presence of the aniline derivative produced in said sample by the reaction of the said enzyme with the said substrate so as to form a pigment in an amount proportional to the amount of the aniline derivative produced in said sample by said enzyme, and quantitatively determining the rate at which the pigment is formed, wherein there is used as the said substrate, a substrate which upon reaction with the said enzyme produces or releases as the said aniline derivative an aniline derivative of the formula (I): 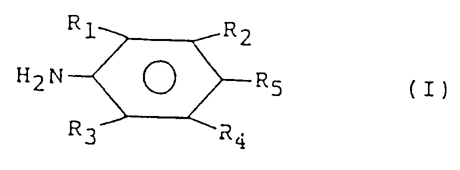
wherein R₁, R₂, R₃ and R₄ may be the same or different and represent hydrogen, halogen, alkyl, sulfo or hydroxyl, R₅ represents hydroxyl, amino or substituted amino, and the substituent represents alkyl, sulfoalkyl or hydroxyalkyl, that aniline derivative serving to increase the rate of oxidation of the chromogen by the said oxidase, the increase of rate being proportional to the activity of the enzyme and to the amount of aniline derivative produced in the sample by the reaction of said enzyme with said substrate and wherein there is used as the chromogen a compound oxidisable by said oxidase in the presence of said aniline derivative to form a pigment which does not react with that aniline derivative to form a condensation product, the enzyme activity being determined from the increase in the rate of formation of the pigment consequent upon the production of said aniline derivative by the said enzyme.

In the method of the present invention, the aniline derivative produced or released by the enzymatic reaction increases the rate at which the chromogen is oxidised by the action of the oxidase in the presence of oxygen. Thus by quantitatively determining the rate at which the pigment is formed in the presence of the aniline derivative the activity of the enzyme can be quantitatively determined. This is based on the finding that a linear relation exists between the amount of the aniline derivative and the rate of formation of the pigment.

Even if the amount of the enzyme contained in a sample is very small, so that the rate of formation of the aniline derivative is slow, the derivative will gradually accumulate in the reaction medium and the rate of formation of the pigment will gradually increase accordingly. By measuring the rate of formation of the pigment the activity of the enzyme contained in a sample can be quantitatively determined.

The enzyme activity of the sample is proportional to the absorbance of the coloured solution containing the pigment, and thus it is advantageous to obtain a calibration curve on the basis of the relation between the enzyme activity and the absorbance.

Quantitative determination of the thus formed pigment can be made according to any one of the known procedures. The determination is simply carried out by measuring the absorbency of the reaction solution coloured by formation of a pigment at the maximum absorption wavelength of the pigment.

As indicated, in the present invention, a specific substrate is used which reacts with the enzyme to produce or release an aniline derivative of formula (I). Typical aniline derivatives of formula (I) are 3,5-dibromo-4-hydroxyaniline (DBHA), 3,5-dichloro-4-hydroxyaniline (DCHA), p-N,N-disulfopropylaminoaniline (SPA), 3,5-diiodo-4-hydroxyaniline (DIHA), 3,3-diaminostilben-4,4'disulfonic acid (DSDA), p-phenylenediamine (PPD), 4-aminoaniline-3-sulfonic acid (DAS), 2-methyl-3,5-dibromo-4-hydroxyaniline (DMBHA), 2,6-dimethyl-3,5-dichloro-4-hydroxyaniline (DMDBHA), 4-N,N-disulfopropylamino-3,5-dibromoaniline (SDBA), 4-(N-ethyl-N-hydroxyethylamino)-3,5-dibromoaniline (EHDBA), 4-N,N-diethylamino-3,4-dihydroxyaniline (DEDHA) and N,N-disulfopropylaniline (DSPA).

When a substrate capable of directly forming an aniline derivative of formula I is not available as a substrate for the enzyme, a conjugate substrate can be used, that is to say a conjugate formed between a known substrate for the enzyme and an aniline derivative of formula (I), that conjugate releasing that derivative when reacted upon by the enzyme. For example, aniline derivatives such as DBHA, DCHA, have a hydroxyl group by means of which they may be linked to a known substrate for the enzyme through an appropriate amino acid or peptide. Typical enzyme and substrate or substrate conjugate are combinations given below:

| **Enzyme** | **Substrate or Substrate Conjugate** |
|---|---|
| γ-GTP | γ-glutamyl-DBHA |
| LAP | L-leucyl-DBHA |
| AAP | L-alanyl-DBHA |
| Cystine aminopeptidase | S-Bz-Cys-DBHA |
| Blood Coagulation Factor X | Bz-Ileu-Glu-Gly-Arg-DBHA |
| Thrombin | D-Phe-Pip-Arg-DBHA |
| Plasmin (from the plasminogen series) | D-Val-Leu-Lys-DBHA |
| Kallikrein | Z-Pro-Phe-Arg-DBHA |
| Chymotrypsin | Bz-Tyr-DBHA |
| Alkali phosphatase | 2,6-dibromo-4-amino-phenyl phosphate |
| N-acetyl glucosaminase | p-aminophenyl-N-acetyl-glucosamine |
| Amylase | 2,6-diiodo-4-aminophenyl-G7 (or G5) |

Any oxidase which can oxidise the chromogen in the presence of oxygen and the aniline derivative to form a pigment, can be used. Preferred oxidases are bilirubin oxidase (BLOD EC 1.3.3.5), monophenol monooxygenase (MPO, EC 1.14.18.1), ascorbate oxidase (AOD, EC 1.10.3.3), catechol oxidase (CAO, EC 1.10.3.1), laccase (EC 1.10.3.2), o-aminophenol oxidase (APO, EC 1.10.3.4), 3-hydroxyanthranilate oxidase (HAO, EC 1.10.3.5) and phenol monooxygenase (PMO, EC1,14,13,7).

Any chromogen which can be oxidised by an oxidase to develop a colour and which does not form a condensation product with the aniline derivative of formula (I) can be used. In order to obtain a higher sensitivity in the determination system, a chromogen with a higher molecular extinction coefficient is preferred. Typical and preferred chromogens are, for example, the compounds P-1 to P-14 represented by the formulae shown in Table 1.

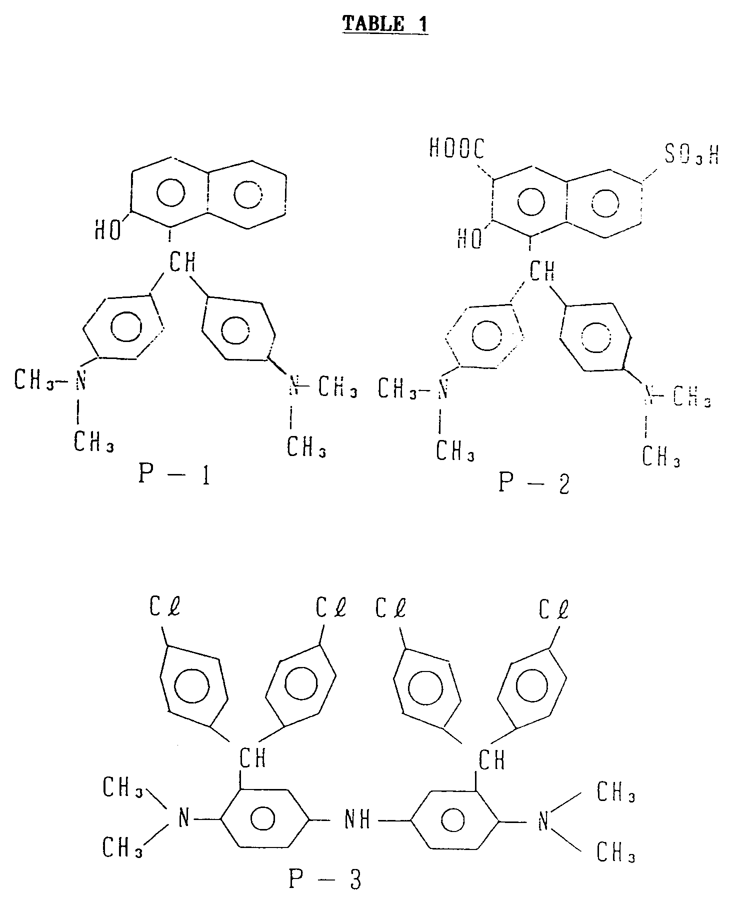

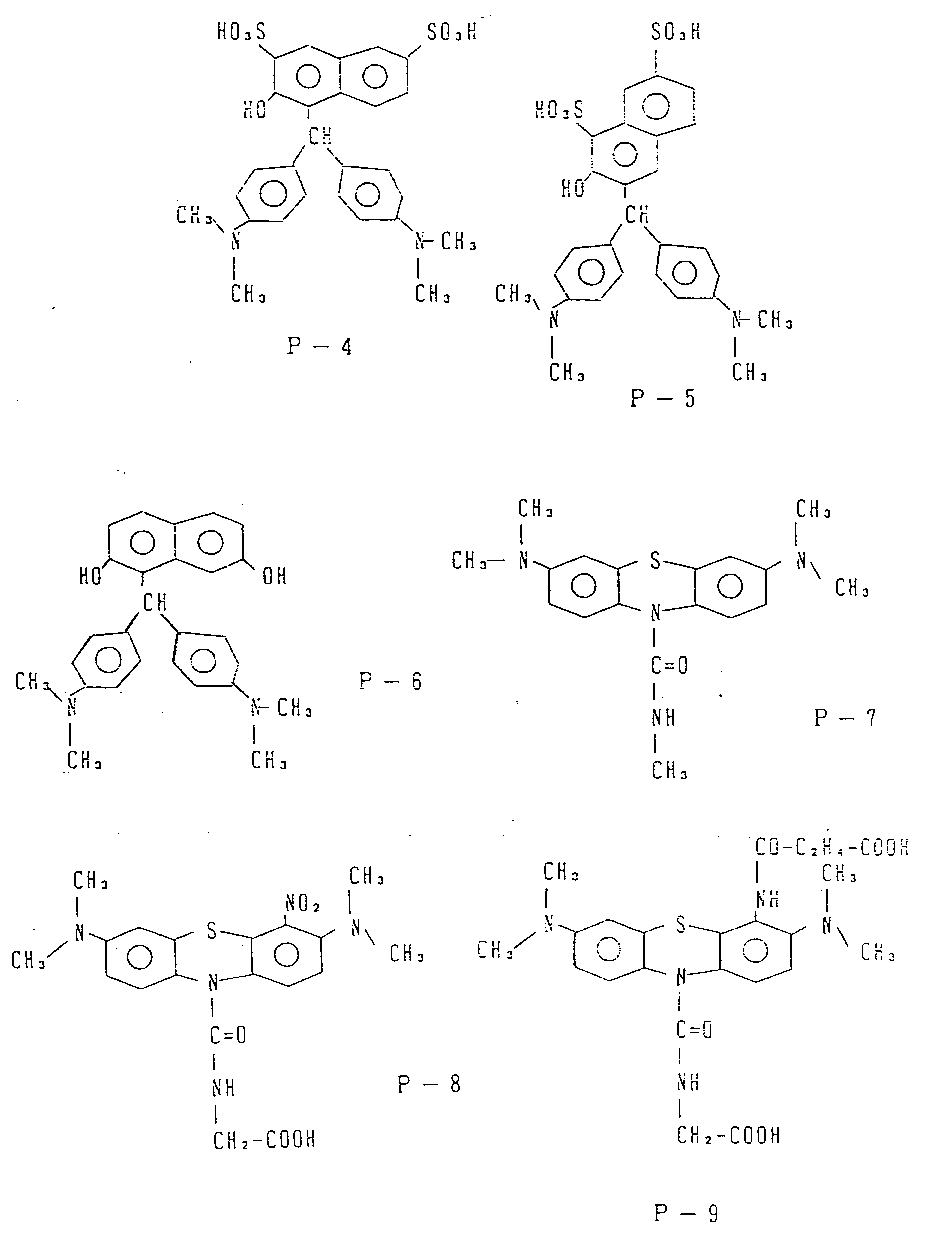

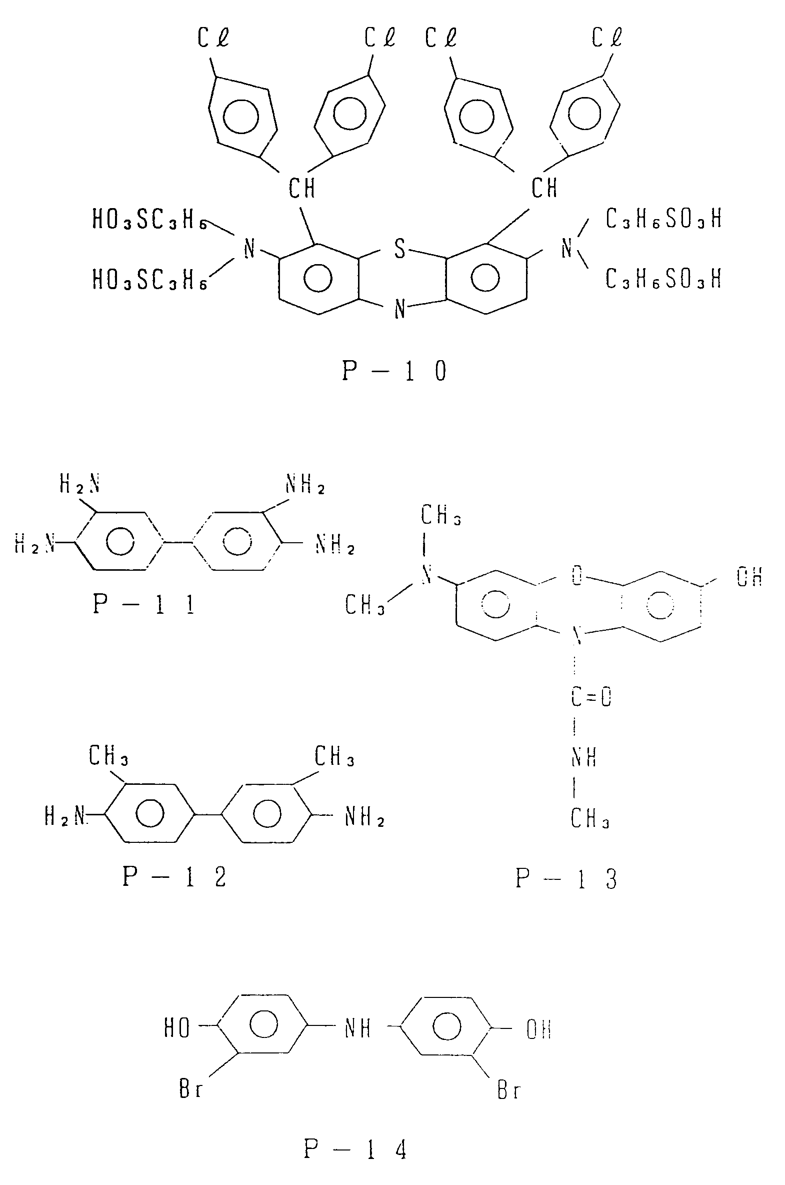

The maximum absorption of these compounds is shown in the following Table 2

**TABLE 2**

| **Chromogen No.** | **Maximum Absorption (nm)** |
|---|---|
| P-1 | 630 |
| P-2 | 630 |
| P-3 | 755 |
| P-4 | 630 |
| P-5 | 630 |
| P-6 | 630 |
| P-7 | 666 |
| P-8 | 655 |
| P-9 | 668 |
| P-10 | 670 |
| P-11 | 435 |
| P-12 | 650 |
| P-13 | 520 |
| P-14 | 590 |

Chromogens P-1, P-2, P-4, P-5 and P-6 are disclosed in EP-A-206316. They are chromogens known as an intermediate for dye synthesis, and are synthesised by a condensation reaction of Michler's hydrol with a naphthalene derivate or by a reduction reaction of a commercially available pigment.

Chromogens P-3 and P-10 are disclosed in EP-A-124287.

Chromogens P-7, P-8, P-9 and P-13, and the preparation thereof are disclosed in EP-B-38205.

Chromogens P-11, P-12 and P-14 are commercially available and can be purchased from Aldrich Co.

In the quantitative determination of enzyme activity according to this invention, a substrate capable of forming or releasing the aniline derivative upon reaction with the enzyme, a chromogen, an oxidase, a buffer reagent and a surfactant, etc. are added to a sample to carry out the enzyme reaction. In the enzymatic reaction, the respective reagents are used at the following concentrations:

| | |
|---|---|
| Buffer reagent | 10 mM- 1M |
| Oxidase | 0.001 - 1,000 U/ml |
| Chromogen | 0.01 - 10 mg/L |
| Substrate | 0.1 - 100 mg/ml |
| Surfactant | 1 - 10 mg/ml |

The reaction is usually carried out at a temperature of 20 - 40°C and at a pH of 5 - 9.

Suitable buffers for use in the present invention include, for example, Good's buffer, phosphate, borate, acetate and tris-hydrochloride.

In another aspect of the present invention, test compositions are provided for determination of enzyme activity by the method of this invention, those compositions containing the enzyme substrate, the chromogen and the oxidase, and preferably the buffer and/or a surfactant.

Also in accordance with this invention diagnostic devices are provided comprising a porous substrate, e.g. a sheet, film or stick of porous material, e.g. filter paper etc. impregnated with a test composition as above described, and which produce a colour response when contacted with a sample, e.g. a urine sample, containing the enzyme, the intensity of that colour response being proportional to the activity of the enzyme in the sample and comparable with that of a blank control to give an evaluation of the enzymatic activity of the samples.

Certain specific embodiments of the invention are illustrated by the following representative examples.

### Example 1

| | |
|---|---|
| DIPSO (Good's buffer solution, made by Dojin Kagaku Kenkyusho) (pH 7.5) | 0.1M |
| Dispanol M-32A (made by Nihon Yushi K.K.) | 5 mg/ml |
| Chromogen P-1 | 0.2 mg/ml |
| BLOD (Bilirubin oxidase) | 0.02 U/ml |
| Gly-Gly | 3 mg/ml |
| MgCl₂ | 1 mg/ml |
| γ-glutamyl-DBHA (G-DBHA) | 2 mg/ml |

To 3.0 ml quantities of the foregoing reagent solution were added 0.02 ml quantities of aqueous γ-GTP solution at concentrations of 2.5, 5, 7.5 and 10 mU/ml respectively. Each mixture was then allowed to stand at 37°C for 30 minutes and changes in the absorbency of the reaction solutions at 630 nm were measured.

The reactions involved are as follows: 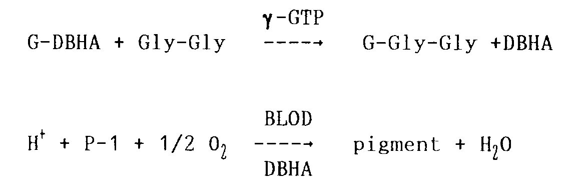
The amount of DBHA in the reaction solution gradually increases with a consequent increase in rate of formation of the pigment. By determining the amount of pigment formed, the activity of γ-GTP in the reaction solution can be determined.

As a control, the same procedure was repeated with a reagent of the same composition as described above, except that 1.5 mg/ml of N-ethyl-N-(3-methylphenyl)-N'-succinyl ethylenediamine (EMSE) was used as a chromogen in place of P-1, and that 0.4 U/ml of BLOD was used. The absorbency of the reaction solution at 710 nm was measured.

The reactions proceed as follows: 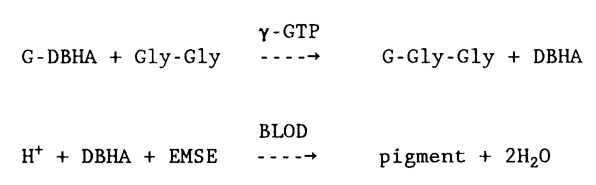
In these reactions the rate of oxidation of the chromogen is not accelerated and the rate of formation of pigment is much slower.

Moreover it is clear from the test results given in Table 3 that the absorbency of the pigment solution is proportional to the concentration of the γ-GTP in the sample.

**TABLE 3**

| γ-GTP (mU/ml) | Aniline Derivative | Absorbency* Chromogen | |
|---|---|---|---|
| | | P-1 | EMSE |
| 2.5 | DBHA | 0.32 | 0.03 |
| 5.0 | DBHA | 0.67 | 0.06 |
| 7.5 | DBHA | 1.02 | 0.09 |
| 10.0 | DBHA | 1.35 | 0.12 |

| | | | |
|---|---|---|---|
| * at 630 nm for P-1; at 710 nm for EMSE | | | |

### Example 2

### Reagent solution A

| | |
|---|---|
| DIPSO Buffer solution (pH7.5) | 0.1 M |
| Dispanol M-32A | 5 mg/ml |
| Chromogen P-2 | 0.2 mg/ml |
| AOD (ascorbic acid oxidase) | 50 U/ml |
| MgCl₂ | 1 mg/ml |
| L-leucyl DSPA | 2 mg/ml |

To 3.0 ml of reagent solution A were added 0.02 ml of an aqueous solution containing 20 mU/ml LAP and the mixture then allowed to stand for 30 minutes. The absorbency of the reaction solution was then measured at 630 nm. As a control, the same procedure was repeated except that EMSE was used in place of P-2, and the absorbency of the reaction solution was measured at 745 nm.

When using the chromogen P-2 the sensitivity of reagent solution A was 5.2 times greater than the control

### Example 3

### Reagent solution B

| | |
|---|---|
| Phosphate buffer solution (pH 7.2) | 0.2 M |
| Triton X-100 | 5 mg/ml |
| Compound P-1 | 1 mg/ml |
| AOD | 200 U/ml |
| γ-glutamyl-DBHA | 10 mg/ml |
| Gly-Gly | 20 mg/ml |

A Wattman No. 41 filter paper, thickness of 0.21 mm, was dipped in solution B and then dried in vacuo. (Triton and Wattman are Registered Trade Marks).

### Reagent solution C

A reagent solution C was prepared having the same composition as solution B, except that 4 mg/ml of EMSE was used in place of Compound P-1, and that 500 U/ml of AOD was used. A test paper was impregnated with solution C and dried as above.

A series of aqueous test solutions were prepared containing 0, 0.1, 0.2, 0.5, 1.0, 2.0, 5.0, 10, 20 or 50 mU/ml of γ-GTP respectively and 100 µl of each test solution was dropped onto each of the test papers B and C to investigate the minimum concentration of γ-GTP that could be detected. At 20 minutes, test paper C could detect γ-GTP down to 5.0 mU/ml, whereas test paper B could detect down to 0.1 mU/ml. At 3 minutes, test paper C could detect γ-GTP down to 50 mU/ml, whereas test paper B could detect down to 1 mU/ml.

When urine of a normal male and urine of a male patient with renal disease were tested on test papers B and C, it was found that the differentiation between the two urine samples required 20 minutes for test paper C, but only 5 minutes with test paper B.

### Example 4

Similar pairs of test papers were prepared using the same solutions and procedures as in Example 3, except that each of oxidases shown in Table 3 was used in place of the AOD used in the test papers of Example 3. Urine from a patient with renal disease was dropped onto each pair of test papers and the colour development on each pair of test papers was compared on a chromatoscanner. The sensitivity ratios for each pair of test papers are shown in Table 4.

**TABLE 4**

| Oxidase | Concentration U/ml | Sensitivity ratio |
|---|---|---|
| MPO | 0.2 | 16.5 |
| CPO | 1.2 | 78.1 |
| Laccase | 0.05 | 4.3 |
| APO | 0.02 | 6.9 |
| HAO | 2.5 | 9.2 |
| PMO | 0.08 | 11.6 |

### Example 5

Example 1 was repeated except that the enzymes and substrates given in Table 5 were used to compare the sensitivity ratios of the reagent solutions according to the invention (A) and the controls (B)

**TABLE 5**

| Enzyme | Substrate | Sensitivity ratio A/B |
|---|---|---|
| CAP | S-Bz-Cys-DBHA | 10.2 |
| X factor | Bz-Ile-Glu-Gly-Arg-DBHA | 14.5 |
| Thrombin | D-Phe-Pip-Arg-DBHA | 9.8 |
| Plasmin | D-Val-Leu-Lys-DBHA | 13.5 |
| Kallikrein | Z-Pro-Phe-Arg-DBHA | 12.6 |

### Example 6

The activities of the enzymes given in Table 7 were determined in the manner described in Example 2, except that the substrates given in the Table were used in place of DSPA, and the sensitivity ratios were obtained in the same way.

**TABLE 7**

| Enzyme | Substrate | Sensitivity ratio A/B |
|---|---|---|
| Alkali phosphatase | 2,6-dichloro-4-aminophenylphosphate | 10.6 |
| N-Acetylglucosaminase | p-aminophenyl-N-acetyl-glucosamine | 4.2 |
| Amylase | 2,6-diiodo-4-amino-phenyl-G7 (or G5) | 2.8 |

### Example 7

Example 2 was repeated except that DIHA, DSDA, PPD, DAS, DMBHA, DMDBHA, SDBA, EHDBA and DEDHA were used separately in place of DSPA. The sensitivity ratios, A/B, were found to be 46.1, 3.4, 6.8, 4.4, 16.6, 20.8, 5.0, 11.4 and 8.9, respectively.

## Claims

1. A method for the chromogenic determination of the activity of an enzyme in a sample, which comprises reacting the sample with a substrate for that enzyme capable of reacting with the enzyme to form an aniline derivative in an amount proportional to the activity of the enzyme, enzymatically oxidising a chromogen by reaction with an oxidase in the presence of the aniline derivative produced in said sample by the reaction of the said enzyme with the said substrate so as to form a pigment in an amount proportional to the amount of aniline derivative produced in said sample by said enzyme, and quantitatively determining the rate at which the pigment is formed, characterised in that there is used as the said substrate, a substrate which upon reaction with the said enzyme produces or releases as the said reaction product an aniline derivative of the formula (I): 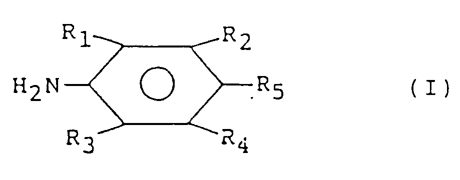 wherein R₁, R₂, R₃ and R₄ may be the same or different and represent hydrogen, halogen, alkyl, sulfo or hydroxyl, R₅ represents hydroxyl, amino or substituted amino, and the substituent represents alkyl, sulfoalkyl or hydroxyalkyl, that aniline derivative serving to increase the rate of oxidation of the chromogen by the said oxidase, that rate of increase being proportional to the activity of the enzyme and to the amount of aniline derivative produced in the sample by the reaction of said enzyme with the said substrate, and wherein there is used as the chromogen, a compound oxidisable by said oxidase in the presence of said aniline derivative to form a pigment which does not react with that aniline derivative to form a condensation product, the enzyme activity being determined from the increase in the rate of formation of the pigment consequent upon the production of said aniline derivative by the said enzyme.

2. A method according to claim 1, wherein the enzyme is γ-glutamyl transpeptidase, leucine aminopeptidase, alanine aminopeptidase, cystine aminopeptidase, blood coagulation factor X, thrombin, plasmin, kallikrein, chymotrypsin, alkali phosphatase, N-acetyl glucosaminase or amylase.

3. A method according to claim 1 or 2, wherein the oxidase is bilirubin oxidase, monophenol monooxygenase, ascorbate oxidase, catechol oxidase, laccase, o-aminophenol oxidase or 3-hydroxyanthranilate oxidase or phenol monooxygenase.

4. A method according to any one of claims 1 to 3, wherein the chromogen is any one of compounds P-1 to P-14 as follows:

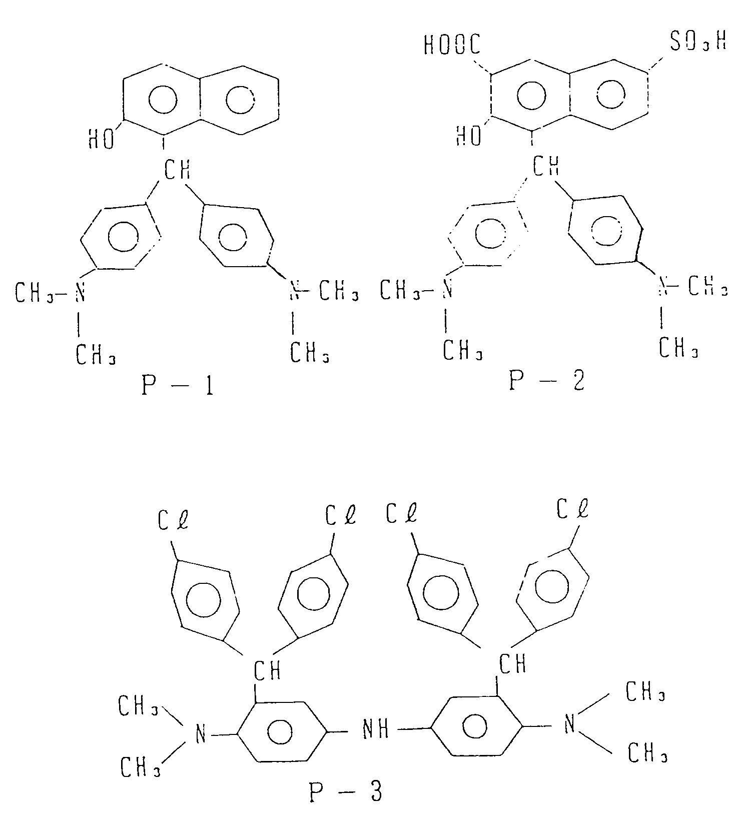

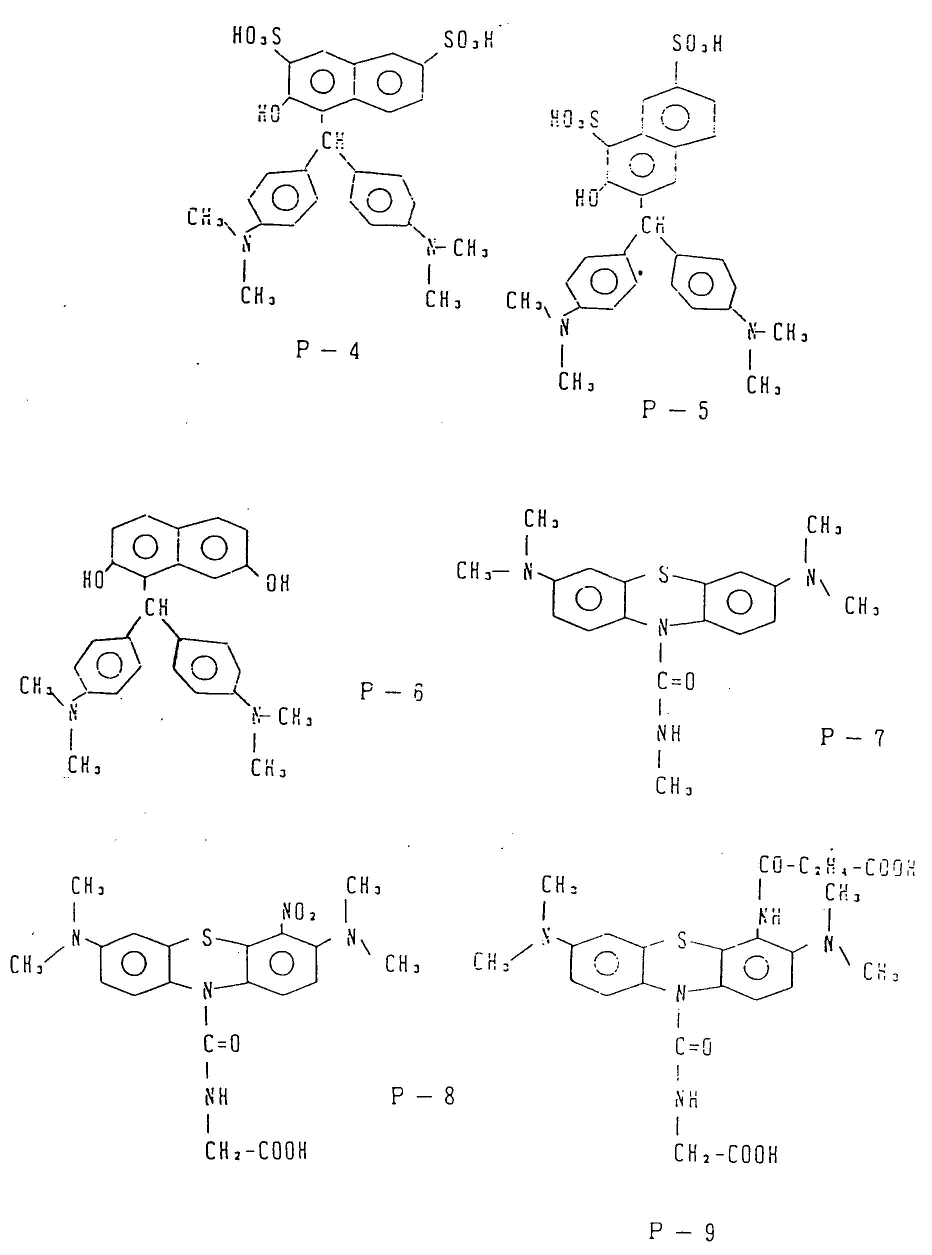

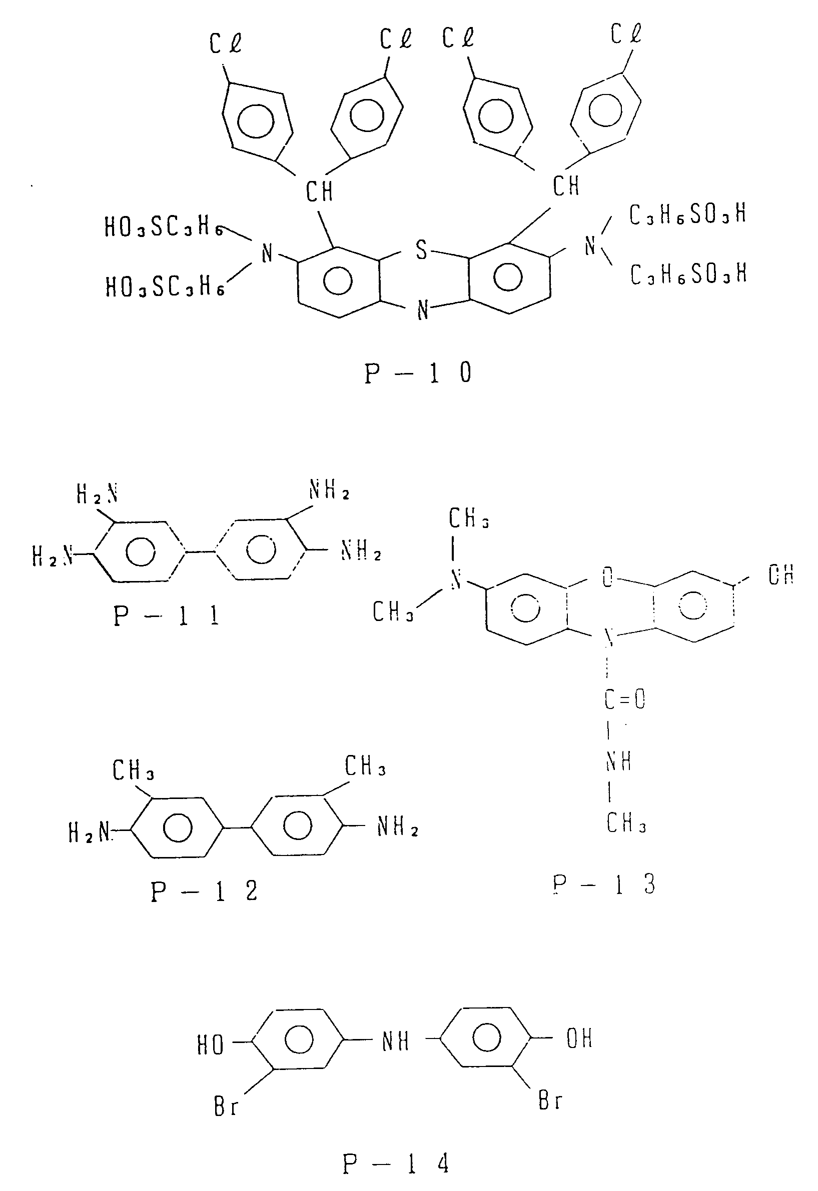

5. A method according to any one of claims 1 to 4, wherein the substrate comprises a conjugate formed between an aniline derivative of formula (I), and a known substrate for the enzyme, the reaction between the enzyme and the substrate conjugate serving to decompose that conjugate and to release that aniline derivative into the sample.

6. A method according to claim 5, wherein the substrate comprises a conjugate formed between an amino acid or peptide, being a substrate for the enzyme whose activity is to be determined, and an aniline derivative of formula (I).

7. A method according to any one of claims 1 to 4, wherein the substrate comprises a compound which itself is converted to an aniline derivative of formula (I) by reaction with the said enzyme.

8. A reagent composition for use in the method of claim 1, that composition comprising a substrate for the enzyme whose activity is to be determined, and being a compound which upon reaction with that enzyme produces an aniline derivative of formula (I), as defined in claim 1, a chromogen oxidisable by an oxidase in the presence of that aniline derivative to form a pigment and an oxidase capable of oxidising the chromogen to form a pigment in the presence of said aniline derivative characterized in that the chromogen and the aniline derivative do not react to form a condensate.

9. A reagent composition according to claim 8, wherein the chromogen is a compound as defined in claim 4.

10. A reagent composition according to claim 8 or 9, wherein the oxidase is as defined in claim 3.

11. A reagent composition according to claim 8, 9 or 10, wherein the enzyme substrate is as defined in claim 5, 6 or 7.

12. A diagnostic device for use in the determination of enzymatic activity by the method of claim 1, comprising a porous substrate material impregnated with a composition as claimed in any one of claims 8 to 11 and which device produces a colour response when contacted with a sample containing the enzyme, the intensity of that colour response being proportional to the activity of the enzyme in the sample.

## Patentansprüche

1. Verfahren zur chromogenen Bestimmung der Aktivität eines Enzyms in einer Probe, umfassend die Umsetzung der Probe mit einem Substrat für das Enzym, das mit dem Enzym reagieren kann, wobei ein Anilinderivat in einer zu der Aktivität des Enzyms proportionalen Menge gebildet wird, die enzymatische Oxidation eines Chromogens durch die Umsetzung mit einer Oxidase in Gegenwart des Anilinderivats, das in der Probe durch die Umsetzung des Enzyms mit dem Substrat gebildet wurde, so daß ein Pigment in einer Menge gebildet wird, die der Menge des in der Probe durch das Enzym gebildeten Anilinderivats proportional ist, und die quantitative Bestimmung der Geschwindigkeit, mit der das Pigment gebildet wird, dadurch gekennzeichnet, daß als Substrat ein Substrat verwendet wird, das bei der Umsetzung mit dem Enzym als Reaktionsprodukt ein Anilinderivat der Formel (I): 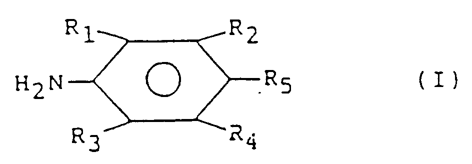 erzeugt oder freisetzt, in der R₁, R₂, R₃ und R₄ gleich oder verschieden sein können und Wasserstoff-, Halogenatome, Alkylreste, Sulfo- oder Hydroxylgruppen bedeuten, R₅ eine Hydroxyl-, Amino- oder substituierte Aminogruppe darstellt, und der Substituent einen Alkyl-, Sulfoalkyl- oder Hydroxyalkylrest bedeutet, wobei das Anilinderivat zur Erhöhung der Geschwindigkeit der Oxidation des Chromogens durch die Oxidase dient, wobei die Geschwindigkeit der Erhöhung zu der Aktivität des Enzyms und zu der durch die Umsetzung des Enzyms mit dem Substrat in der Probe gebildeten Menge des Anilinderivats proportional ist, und wobei als Chromogen eine Verbindung verwendet wird, die in Gegenwart des Anilinderivats durch die Oxidase oxidiert werden kann, wobei ein Pigment gebildet wird, das mit dem Anilinderivat nicht unter Bildung eines Kondensationsproduktes reagiert, wobei die Enzymaktivität aus der Erhöhung der Bildungsgeschwindigkeit des Pigments bestimmt wird, die sich aus der Bildung des Anilinderivats durch das Enzym ergibt.

2. Verfahren nach Anspruch 1, wobei das Enzym γ-Glutamyltranspeptidase, Leucinaminopeptidase, Alaninaminopeptidase, Cystinaminopeptidase, der Blutgerinnungsfaktor X, Thrombin, Plasmin, Kallikrein, Chymotrypsin, alkalische Phosphatase, N-Acetylglucosaminase oder Amylase ist.

3. Verfahren nach Anspruch 1 oder 2, wobei die Oxidase Bilirubinoxidase, Monophenolmonooxygenase, Ascorbatoxidase, Catechinoxidase, Laccase, o-Aminophenoloxidase oder 3-Hydroxyanthranilatoxidase oder Phenolmonooxygenase ist.

4. Verfahren nach einem der Ansprüche 1 bis 3, wobei das Chromogen eine der nachstehenden Verbindungen P-1 bis P-14 ist:

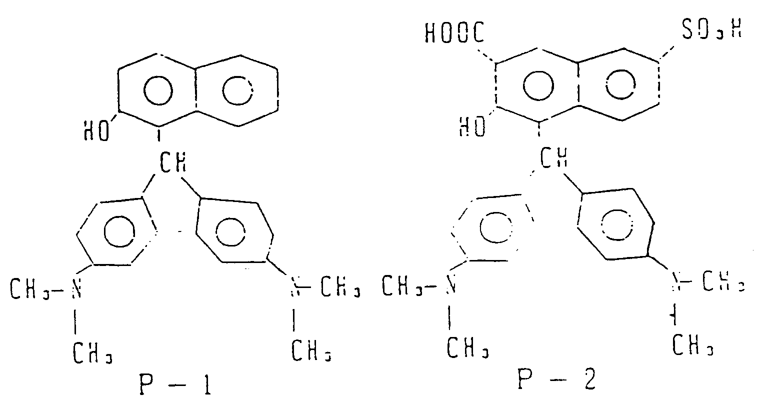

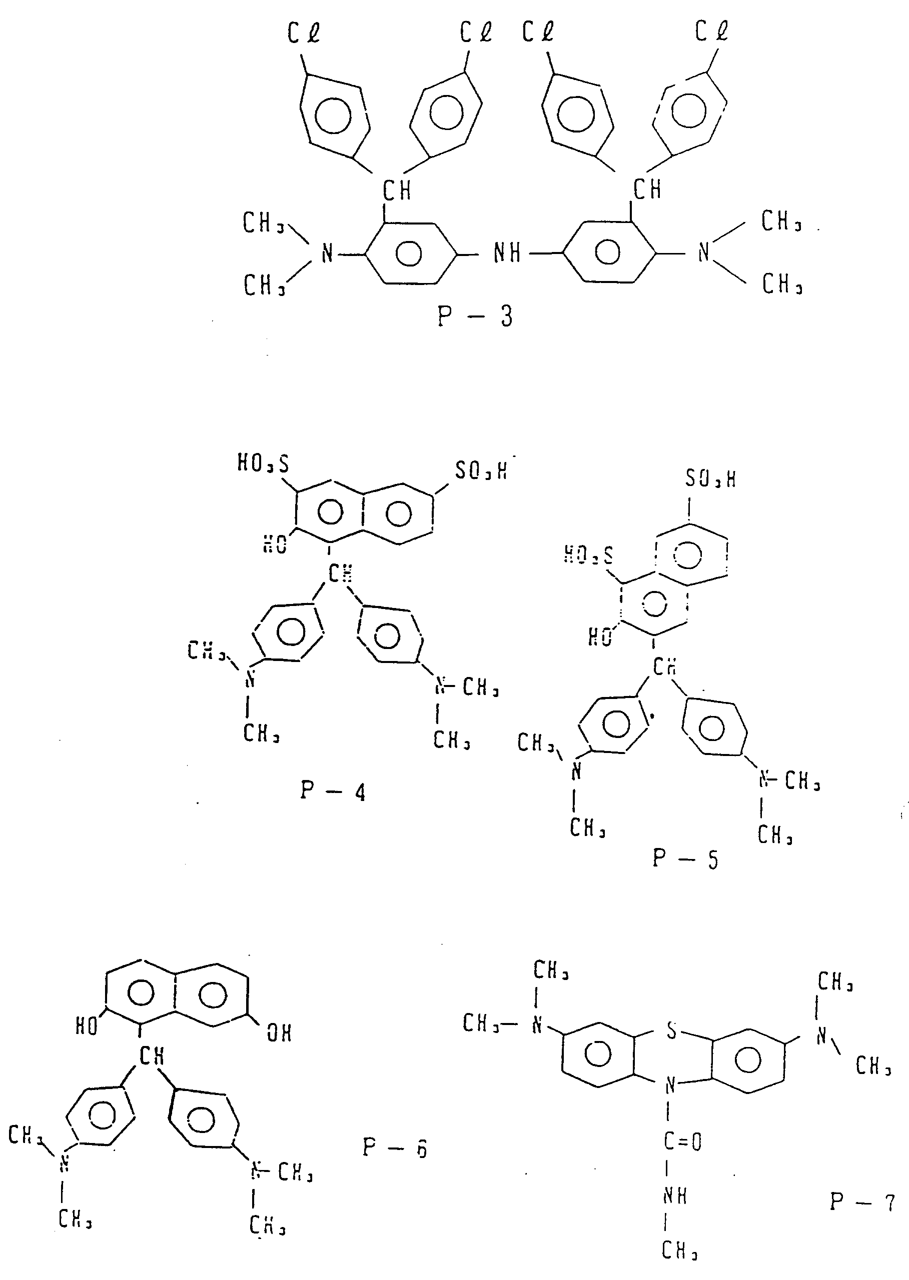

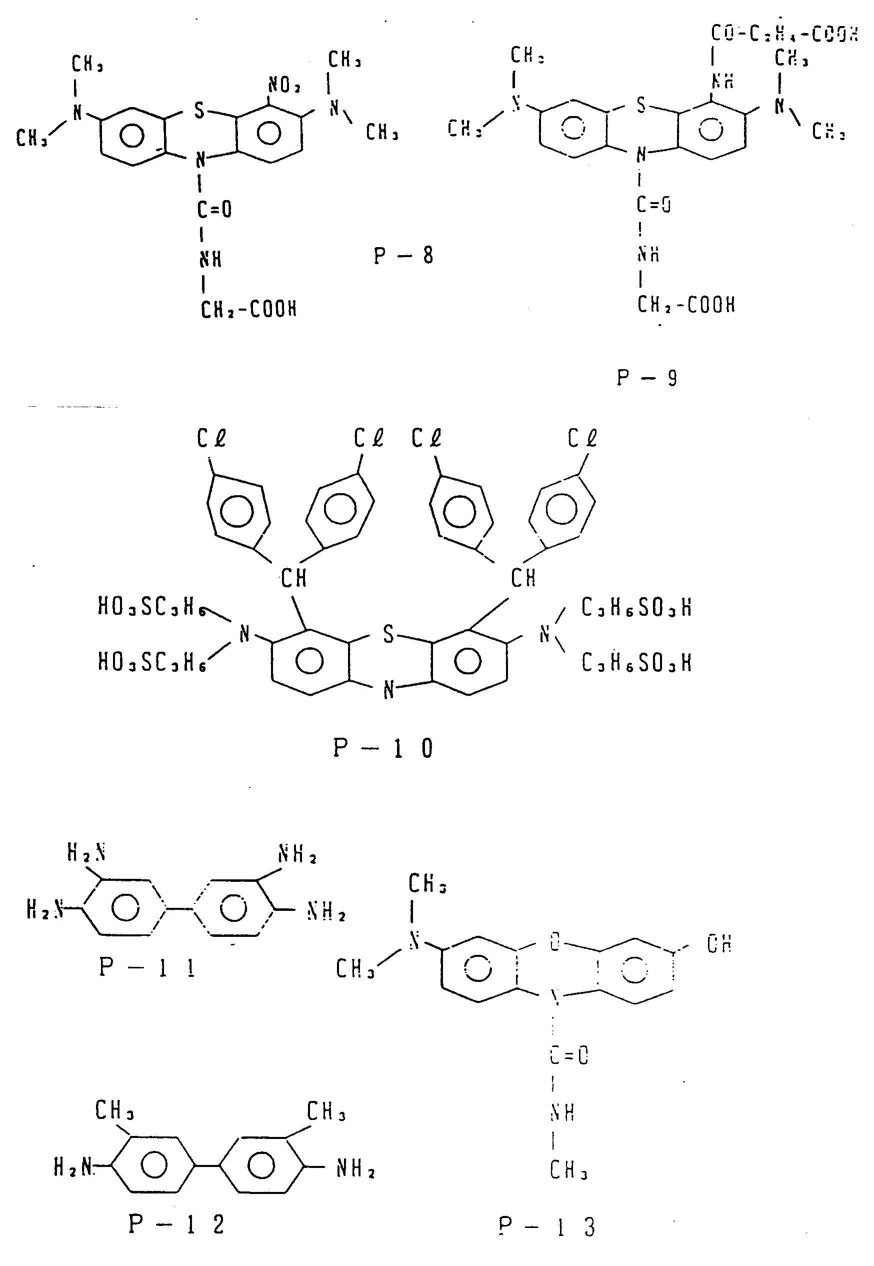

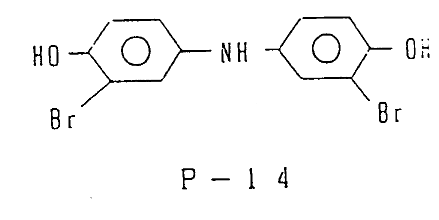

5. Verfahren nach einem der Ansprüche 1 bis 4, wobei das Substrat ein Konjugat umfaßt, das zwischen einem Anilinderivat der Formel (I) und einem bekannten Substrat für das Enzym gebildet wird, und die Umsetzung zwischen dem Enzym und dem Substratkonjugat zur Spaltung des Konjugats und zur Freisetzung des Anilinderivats in die Probe dient.

6. Verfahren nach Anspruch 5, wobei das Substrat ein zwischen einer Aminosäure oder einem Peptid, das ein Substrat für das Enzym ist, dessen Aktivität bestimmt werden soll, und einem Anilinderivat der Formel (I) gebildetes Konjugat umfaßt.

7. Verfahren nach einem der Ansprüche 1 bis 4, wobei das Substrat eine Verbindung umfaßt, die selbst durch die Umsetzung mit dem Enzym in ein Anilinderivat der Formel (I) umgewandelt wird.

8. Reagenszusammensetzung zur Verwendung in dem Verfahren von Anspruch 1, wobei die Zusammensetzung ein Substrat für das Enzym, dessen Aktivität bestimmt werden soll, und das eine Verbindung ist, die bei der Umsetzung mit dem Enzym ein Anilinderivat der Formel (I) bildet, wie in Anspruch 1 definiert, ein Chromogen, das in Gegenwart des Anilinderivats durch eine Oxidase oxidiert werden kann, wobei ein Pigment gebildet wird, und eine Oxidase umfaßt, die das Chromogen oxidieren kann, wobei in Gegenwart des Anilinderivates ein Pigment gebildet wird, dadurch gekennzeichnet, daß das Chromogen und das Anilinderivat nicht unter Bildung eines Kondensats reagieren.

9. Reagenszusammensetzung nach Anspruch 8, wobei das Chromogen eine wie in Anspruch 4 definierte Verbindung ist.

10. Reagenszusammensetzung nach Anspruch 8 oder 9, wobei die Oxidase wie in Anspruch 3 definiert ist.

11. Reagenszusammensetzung nach Anspruch 8, 9 oder 10, wobei das Enzymsubstrat wie in Anspruch 5, 6 oder 7 definiert ist.

12. Diagnostische Vorrichtung zur Verwendung bei der Bestimmung der Enzymaktivität durch das Verfahren von Anspruch 1, umfassend ein poröses Substratmaterial, das mit einer Zusammensetzung nach einem der Ansprüche 8 bis 11 imprägniert ist, und wobei die Vorrichtung eine Farbantwort erzeugt, wenn sie mit einer das Enzym enthaltenden Probe in Kontakt gebracht wird, wobei die Intensität der Farbantwort der Aktivität des Enzyms in der Probe proportional ist.

## Revendications

1. Procédé de détermination, grâce à la formation d'une couleur, de l'activité d'une enzyme dans un échantillon, lequel procédé comporte le fait de faire réagir l'échantillon avec un substrat de l'enzyme, capable de réagir avec cette enzyme de façon à produire un dérivé d'aniline en une quantité proportionnelle à l'activité de l'enzyme, le fait de réaliser l'oxydation enzymatique d'un agent chromogène en faisant réagir celui-ci avec une oxydase, en présence du dérivé d'aniline produit dans ledit échantillon par la réaction de ladite enzyme avec ledit substrat, de façon à former un pigment en une quantité proportionnelle à la quantité du dérivé d'aniline produit dans ledit échantillon par ladite enzyme, et le fait de déterminer quantitativement la vitesse à laquelle le pigment est formé, procédé caractérisé en ce que l'on utilise, en tant que ledit substrat, un substrat qui, en réagissant avec ladite enzyme, produit ou libère, en tant que produit de ladite réaction, un dérive d'aniline de formule (I): 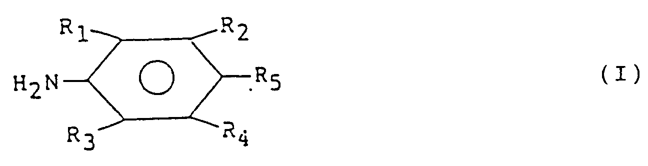 dans laquelle R₁, R₂, R₃ et R₄ peuvent être identiques ou différents et représentent chacun un atome d'hydrogène ou d'halogène ou un groupe alkyle, sulfo ou hydroxy, et R₅ représente un groupe hydroxy, amino ou amino substitué, le substituant étant un groupe alkyle, sulfoalkyle ou hydroxyalkyle, ce dérivé d'aniline servant à augmenter la vitesse d'oxydation de l'agent chromogène par ladite oxydase, l'augmentation de cette vitesse étant proportionnelle à l'activité de l'enzyme et à la quantité de dérivé d'aniline produite dans l'échantillon par la réaction de ladite enzyme avec ledit substrat, et dans lequel procédé on utilise comme agent chromogène un composé qui peut être oxydé par ladite oxydase en présence dudit dérivé d'aniline de façon à former un pigment et qui ne réagit pas avec ce dérivé d'aniline pour former un produit de condensation, l'activité de l'enzyme étant déterminée à partir de l'augmentation de la vitesse de formation du pigment consécutive à la production dudit dérivé d'aniline par ladite enzyme.

2. Procédé conforme à la revendication 2, dans lequel l'enzyme est la γ-glutamyl-transpeptidase, la leucine aminopeptidase, l'alanine aminopeptidase, la cystine aminopeptidase, le facteur X de coagulation du sang, la thrombine, la plasmine, la kallikréine, la chymotrypsine, la phosphatase alcaline, la N-acétyl-glucosaminase ou l'amylase.

3. Procédé conforme à la revendication 1 ou 2, dans lequel l'oxydase est la bilirubine oxydase, la monophénol monooxygénase, l'ascorbate oxydase, la catéchol oxydase, la laccase, l'orthoaminophénol oxydase, la 3-hydroxyanthranilate oxydase ou la phénol monooxygénase.

4. Procédé conforme à l'une des revendications 1 à 3, dans lequel l'agent chromogène est l'un quelconque des composés P-1 à P-14 indiqués ci-dessous :

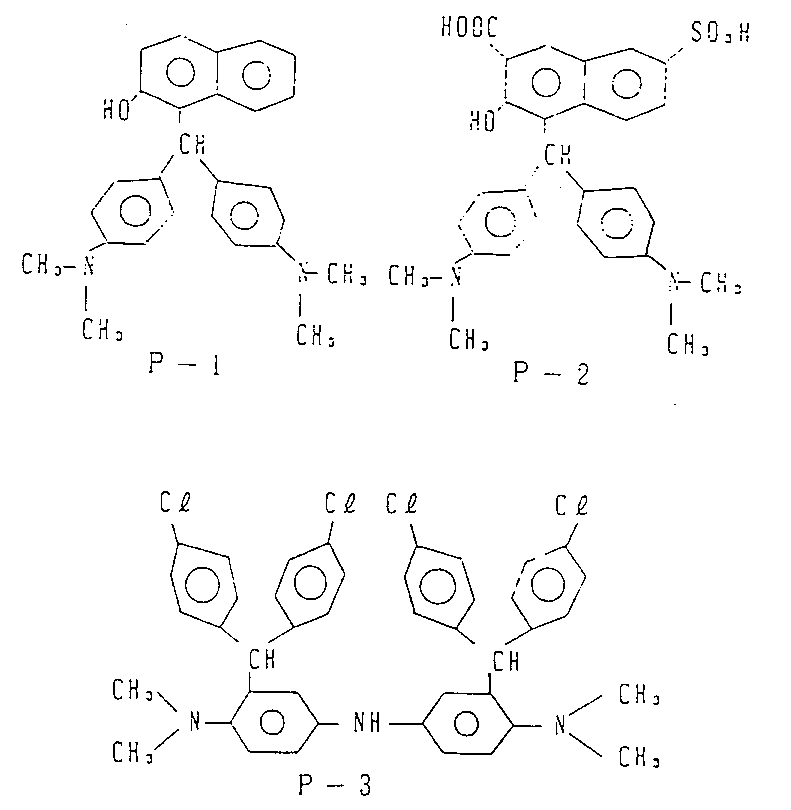

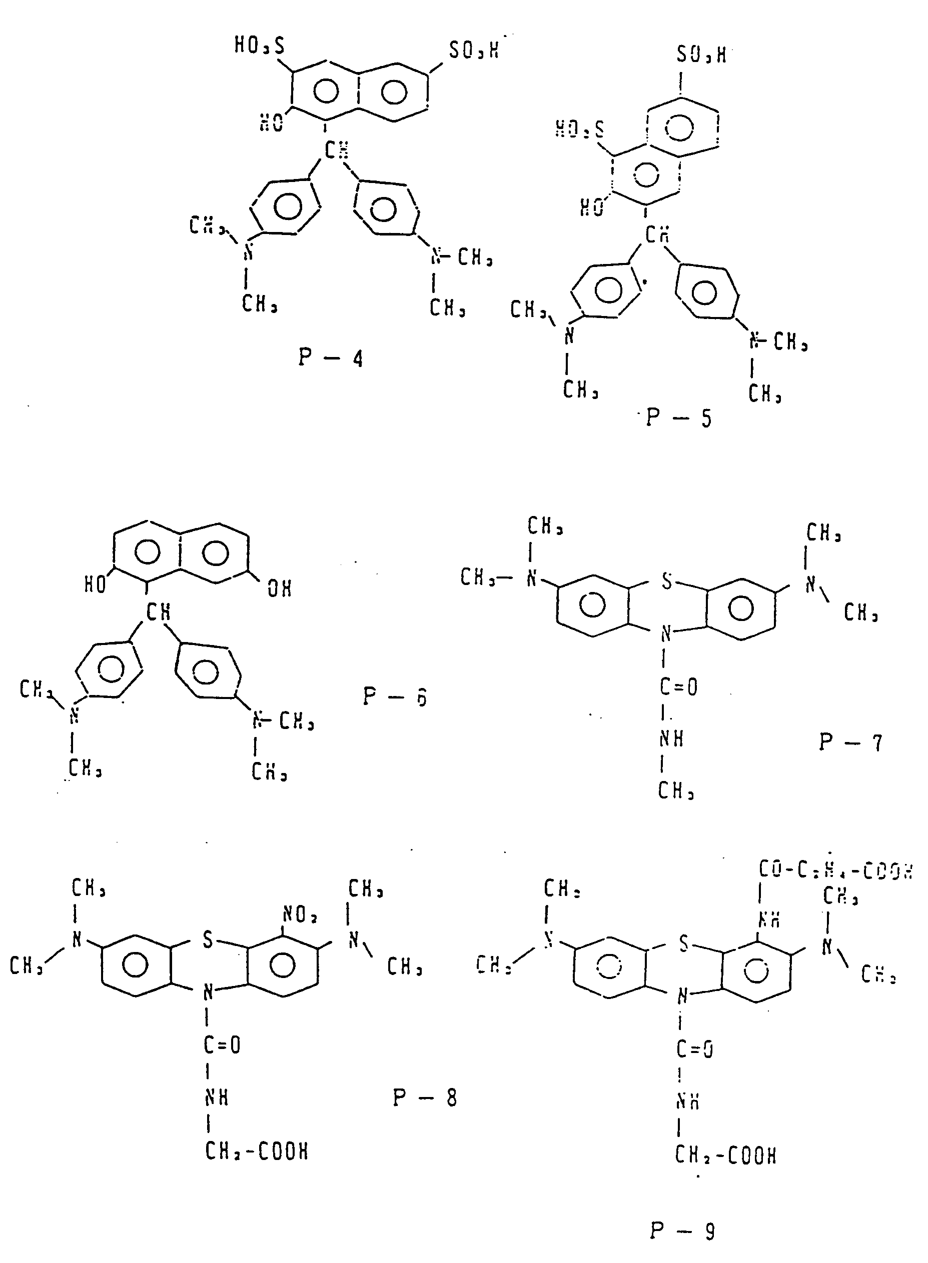

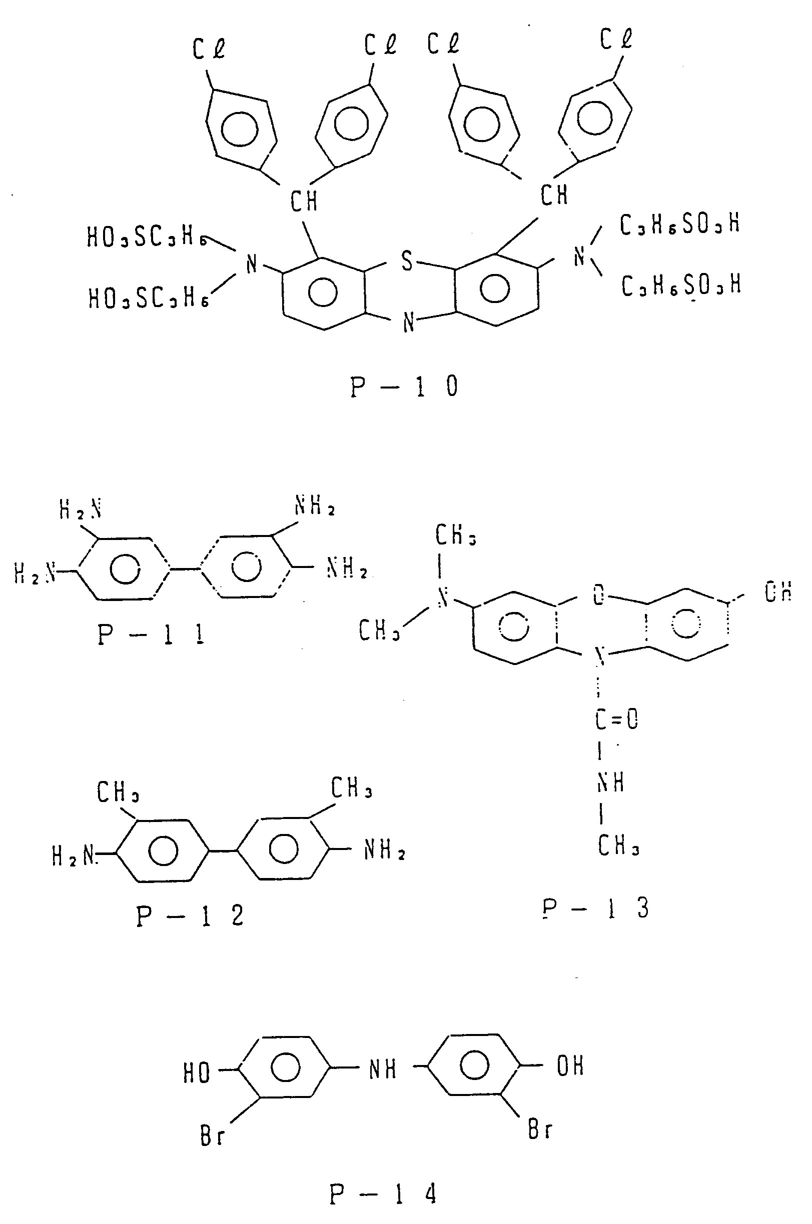

5. Procédé conforme à l'une des revendications 1 à 4, dans lequel le substrat est constitué par un conjugué formé entre un dérivé d'aniline de formule (I) et un substrat connu de l'enzyme, la réaction entre l'enzyme et le conjugué de substrat servant à décomposer ce conjugué et à libérer le dérivé d'aniline dans l'échantillon.

6. Procédé conforme à la revendication 5, dans lequel le substrat est constitué d'un conjugué formé entre un acide aminé ou un peptide, qui est un substrat de l'enzyme dont on veut déterminer l'activité, et un dérivé d'aniline de formule (I).

7. Procédé conforme à l'une des revendications 1 à 4, dans lequel le substrat est constitué d'un composé qui est lui-même converti en un dérivé d'aniline de formule (I) par réaction avec ladite enzyme.

8. Composition de réactifs destinée à être utilisée dans le procédé conforme à la revendication 1, cette composition comportant un substrat de l'enzyme dont on veut déterminer l'activité, qui est un composé qui, à la suite d'une réaction avec cette enzyme, donne un dérivé d'aniline de formule (I), défini dans la revendication 1, un agent chromogène qui peut être oxydé par une oxydase, en présence de ce dérivé d'aniline, pour former un pigment, et une oxydase capable d'oxyder l'agent chromogène pour former un pigment, en présence dudit dérivé d'aniline, caractérisée en ce que l'agent chromogène et le dérivé d'aniline ne réagissent pas pour former un produit de condensation.

9. Composition de réactifs conforme à la revendication 8, dans laquelle l'agent chromogène est un compose défini dans la revendication 4.

10. Composition de réactifs conforme à la revendication 8 ou 9, dans laquelle l'oxydase est une oxydase définie dans la revendication 3.

11. Composition de réactifs conforme à la revendication 8, 9 ou 10, dans laquelle le substrat de l'enzyme est un substrat défini dans la revendication 5, 6 ou 7.

12. Dispositif de diagnostic, destiné à être utilisé pour déterminer une activité enzymatique selon le procédé conforme à la revendication 1, qui comporte un matériau de substrat poreux, imprégné d'une composition conforme à l'une des revendications 8 à 11, lequel dispositif fait apparaître une couleur, en guise de réponse, lorsqu'il est mis en contact avec un échantillon contenant l'enzyme, l'intensité de cette couleur apparue en réponse étant proportionnelle à l'activité de l'enzyme dans l'échantillon.
